# EUROPÄISCHE PATENTSCHRIFT

(11) **EP 2 203 658 B1**
(45) Veröffentlichungstag und Bekanntmachung des Hinweises auf die Patenterteilung: **09.03.2011**
(21) Anmeldenummer: 08804127.2
(22) Anmeldetag: 12.09.2008
(51) Int. Cl.: F16D 65/14, F16C 41/00, F16D 55/00

(54) **WÄLZLAGER MIT EINER BREMSEINRICHTUNG**
ROLLER BEARING HAVING A BRAKE DEVICE
PALIER À ROULEMENT AVEC UN DISPOSITIF DE FREINAGE

(30) Priorität: 26.10.2007 DE 102007051229
(43) Veröffentlichungstag der Anmeldung: 07.07.2010
(73) Patentinhaber: Schaeffler Technologies GmbH & Co. KG, 91074 Herzogenaurach (DE)
(72) Erfinder: NUISSL, Christian, 90439 Nürnberg (DE); STÖLZLE, Jürgen, 91056 Erlangen (DE)
(86) Internationale Anmeldenummer: PCT/EP2008/062164
(87) Internationale Veröffentlichungsnummer: WO 2009/053168

(56) Entgegenhaltungen:
- DE-A1- 10 007 317
- DE-A1- 10 127 487
- US-A- 3 752 267

## Beschreibung

### Gebiet der Erfindung

Die Erfindung betrifft ein Wälzlager mit einer Bremseinrichtung, insbesondere Drehverbindung, bestehend aus einem Lageraußenring und einem Lagerinnenring, zwischen denen auf zugehörigen Laufbahnen Wälzkörper abrollen, wobei zur Erzeugung einer Bremswirkung durch Reibschluss ein mit einem der Lagerringe verbundenes verschiebbares Bremselement gegen eine mit dem zugehörigen anderen Lagerring verbundene Gegenfläche gepresst ist und der Reibschluss mit Hilfe eines Elektromagneten aufhebbar ist.

### Hintergrund der Erfindung

Wälzlager mit Bremseinrichtungen sind bereits seit längerem bekannt. So besteht bei Wälzlagerdrehverbindungen an Windkraftanlagen die Gefahr, dass sie nach relativ kurzer Zeit durch Riffelbildung in den Laufbahnen ausfallen. Dieses Phänomen wird insbesondere durch geringe Schwenkbewegungen zum Ausgleich der Windrichtung erzeugt, bei der es zu einem Gleiten der Wälzkörper auf der Laufbahn kommt. Um diesen Verschleiß auszuschalten, ist es bekannt, durch verschiedene Maßnahmen den geringen Drehwiderstand bei Wälzlagern zu erhöhen. Die DE 37 25 972 A1 und die DE 41 04 137 A1 schlagen in diesem Zusammenhang vor, eine zusätzlich umlaufende Bremseinrichtung einzusetzen. Die Bremskraft und somit der gewünschte Drehwiderstand kann dann von außen eingestellt werden. Nachteilig dabei ist, dass im ersten Fall das Bremselement nur bei Stilllegung der Windkraftanlage aufgehoben werden kann. Im zweiten Fall besteht die Bremseinrichtung aus vielen mechanischen Einzelteilen und ist dadurch aufwändig zu fertigen und kompliziert in der Handhabung.

Aus der DE 19 04 954 B ist eine drehzapfenlose Drehverbindung für Bagger, Krane oder dergleichen zur Lagerung eines schwenkbaren Oberbaus auf einem Unterbau bekannt. Diese Drehverbindung besteht jeweils aus einem einteiligen Drehring sowie einem zweiteiligen, aus zwei Profilringen zusammengesetzten weiteren Drehring. Die beiden Drehringe sind jeweils durch die Kugeln eines zweireihigen Kugellagers gegeneinander abgestützt und mit einer Bremsvorrichtung ausgerüstet. Die Bremsvorrichtungen weisen jeweils einen oder mehrere Bremsklotzträger auf, die an einem mit dem einteiligen Drehring in Verbindung stehenden Bauteil befestigt sind. Bei dieser Anordnung ist von Nachteil, dass die Bremsvorrichtungen außerhalb der eigentlichen Lageranordnung angeordnet sind und daher zusätzlichen Bauraum beanspruchen.

Eine gattungsgemäße Lageranordnung mit Bremsfunktion ist aus der DE 101 27 487 A1 vorbekannt. Die Radiallageranordnung gemäß Figur 1 weist ein als Radiallager ausgebildetes Rillenkugellager auf und eine axial daneben angeordnete Bremseinrichtung. Das Rillenkugellager besteht aus dem Innenring, dem Außenring und zwischen beiden in einem Käfig angeordneten Lagerkugeln. Weiterhin verfügt das Rillenkugellager über zwei Dichtringe, die den Ringraum beidseitig gegen die Umgebung abdichten. Die Bremseinrichtung weist einen inneren Haltering und äußeren Haltering auf. An einem radial nach außen gerichteten Flansch des inneren Halteringes ist die über eine Flachdrahtfeder eine Bremsscheibe befestigt, die aus einem ferromagnetischen Material besteht und auf ihrer vom Flansch abgewandten Seite einen Bremsbelag besitzt. Durch die Befestigung mittels der Flachdrahtfeder ist die Bremsscheibe drehfest mit dem inneren Haltering verbunden und in Axialrichtung verschiebbar. Gegenüber dem Bremsbelag ist am äußeren Haltering eine Gegenfläche ausgebildet, gegen die der Bremsbelag beim Bremsen gepresst wird. Der äuβere Haltering weist weiterhin eine elektrische Spule und einen oder mehrere Permanentmagneten auf, die jeweils im Bereich zwischen der Bremsscheibe und dem Rillenkugellager angeordnet und mechanisch mit dem äußeren Haltering und somit auch mit der Gegenfläche verbunden sind.

Nachteilig dabei ist, dass die Bremseinrichtung in axialer Richtung als externes Teil an das Lager angeflanscht werden muss und daher zusätzlichen Bauraum beansprucht. Die Halteringe sind relativ kompliziert aufgebaut und müssen erst in aufwendiger Weise durch Stifte mit den Lagerringen verbunden werden. Ein weiterer Nachteil ist dadurch begründet, dass die Bremswirkung durch einen Permanentmagneten ausgelöst wird, der die Bremsscheibe anzieht. Bei bestimmten Anwendungsfällen ist aber ein stetiges Magnetfeld nachteilig, da unter Umständen eisenhaltiger Schmutz vom Lager angezogen wird. Außerdem ist bei den vorstehend beschriebenen Bremseinrichtungen von Nachteil, dass diese für bestimmte Anwendungsfälle eine zu geringe Bremskraft entwickeln.

### Zusammenfassung der Erfindung

Der Erfindung liegt daher die Aufgabe zugrunde, die genannten Nachteile zu vermeiden und eine einfach zu fertigende Bremsvorrichtung bereitzustellen, die bei einem minimalen Bauraum eine hohe Bremsleistung entwickelt.

Erfindungsgemäß wird diese Aufgabe nach dem kennzeichnenden Teil von Anspruch 1 in Verbindung mit dessen Oberbegriff dadurch gelöst, dass der Elektromagnet aus einem der Lagerringe als Weicheisenkem und einer diesen umschließenden Spule besteht, dass eine mit einem der Lagerringe verbundene ferromagnetische Ankerplatte in Richtung einer mit dem anderen Lagerring verbundene Druckplatte gepresst ist und zwischen Ankerplatte und Druckplatte wenigstens je eine Scheibe angeordnet ist, die beidseitig mit Bremsbelägen in Wirkverbindung stehen und über getrennt ausgebildete Führungsstifte mit je einem der Lagerringe formschlüssig verbunden sind.

Auf diese Weise ist eine modular aufgebaute Bremseinrichtung geschaffen, deren Haltedrehmoment bei gleichem radialen Bauraum um ein ungeradzahliges Vielfaches, je nach Anzahl der zwischen Ankerplatte und Druckplatte angeordneten Scheiben, erhöht ist. Die einzelnen Scheiben sind dabei abwechordneten Scheiben, erhöht ist. Die einzelnen Scheiben sind dabei abwechselnd drehend und nicht drehend ausgebildet, sodass sich eine Relativbewegung zwischen diesen einstellt. Diese erfolgt durch Führungsstifte, welche eine axiale Verschiebung und eine rotative Mitnahme ermöglichen, wobei die Scheiben abwechselnd mit dem Lagerinnenring und dem Lageraußenring verbunden sind.

Durch Unterbringen des Bremselementes in einem der Lagerringe, im Normalfall im sich drehenden Lagerring, wird es auf diese Weise möglich, dass Wälzlager mit Bremseinrichtung raumsparend zu realisieren. Ein weiterer Vorteil liegt darin, dass durch die Anordnung der Bremseinrichtung als integraler Wälzlagerbestandteil diese nicht in zusätzlicher und aufwändiger Weise mit der eigentlichen Lageranordnung zu verbinden ist. Es ist auch von Vorteil, dass durch den Einsatz unterschiedlich dimensionierter Federn in einfacher Weise auf die Größe der Vorspannkraft und damit auf die aufzubringende Bremskraft eingewirkt werden kann. Auch kann durch den Einsatz des Elektromagneten die Bremskraft problemlos aufgehoben werden, sodass in diesem Fall das Wälzlager leichtgängig bewegt werden kann. Ein derart gattungsgemäß ausgebildetes Wälzlager mit Bremseinrichtung lässt sich immer besonders dann vorteilhaft einsetzen, wenn ein stetig vorhandenes Reibmoment erwünscht ist, aber auch unter bestimmten Umständen sehr schnell ein Lösen dieses Reibmoments erzielt werden muss. Dies ist beispielsweise im medizinischen Bereich der Fall, wenn die Wälzlageranordnung in einer Drehverbindung eingesetzt wird, beispielsweise in einem Deckenstativ, das mit unterschiedlich ausgestalteten medizinischen Geräten verbunden ist. Dabei ist von Vorteil, dass durch das stetig vorhandene Reibmoment ein unerwünschtes Verdrehen der Drehverbindung einerseits unterbunden wird, andererseits die Drehverbindung aber durch Lösen der Bremseinrichtung leicht verstellbar ist.

Weitere vorteilhafte Ausgestaltungen der Erfindung sind in den Unteransprüchen beschrieben.

Nach Anspruch 2 ist vorgesehen, dass die Ankerplatte über mehrere in Umfangsrichtung voneinander beabstandete Führungsstifte axial verschiebbar gehalten und über mehrere in Umfangsrichtung voneinander beabstandete Federelemente mit einer Vorspannung beaufschlagt ist, wobei im stromlosen Zustand der Spule zwischen der Ankerplatte und dem Lagerring ein Luftspalt gebildet ist. Auf diese Weise sind die die Bremswirkung auslösenden Mittel, nämlich die Federelemente, und die die Bremswirkung aufhebenden Mittel, nämlich die Spulenwicklungen, unmittelbar benachbart im Lagerring angeordnet, sodass der vorhandene Bauraum maximal ausgelastet ist.

Nach einem weiteren Merkmal gemäß Anspruch 3 soll die Druckplatte kreisringartig ausgebildet sein und über ein Gewinde von einem zugehörigen Gewinde des Lagerringes aufgenommen sein. Dadurch ist sichergestellt, dass der Luftspalt des Elektromagneten zur Aufhebung der Bremskraft sehr genau einstellbar ist. Ist der Luftspalt zu gering eingestellt, so besteht die Gefahr, dass die Bremswirkung nicht aufgehoben wird, da der Bremsbelag nicht abhebt. Ist der Luftspalt hingegen zu groß, so wird das Magnetfeld geschwächt und der Elektromagnet muss unnötig groß ausgelegt werden.

Nach einem anderen zusätzlichen Merkmal gemäß Anspruch 4 ist vorgesehen, dass die Wälzkörper durch Lagernadeln zweier entgegengerichteter Axialschrägnadellager gebildet sind, wobei ein Schnittpunkt von deren verlängerter Drehachsen im Lagerinnenring oder im Lageraußenring liegt. Gegenüber den bekannten Drehverbindungen, die bevorzugt als Vierpunktlager oder als Kreuzrollenlager ausgeführt sind, ist bei Verwendung von zwei Axialschrägnadellagern bei gleicher oder höherer Tragzahl die Fertigung wesentlich kostengünstiger. In diesem Zusammenhang hat es sich nach Anspruch 5 als vorteilhaft erwiesen, dass die Axialschrägnadellager zueinander in O-Anordnung angestellt sind und die Laufbahnen tragende Laufscheiben aufweisen. Diese können dann nach einem weiteren Merkmal der Erfindung gemäß Anspruch 6 einem Härteprozess unterworfen werden, wobei es sich als nach Anspruch 7 als vorteilhaft erwiesen hat, dass Laufscheiben und wenigstens einer der Lagerringe aus unterschiedlichen Werkstoffen bestehen können, sodass nochmals eine Gewichtsreduzierung der genannten Lageranordnung Gewichtsreduzierung der genannten Lageranordnung realisierbar ist. Vorteilhaft ist es in diesem Fall nach Anspruch 8, wenn der spulenlose Lagerring aus einem Leichtmetall oder aus einem Kunststoff gefertigt ist, der die wesentlich härteren Laufscheiben der Axialschrägnadellager aufnimmt.

Nach einem anderen zusätzlichen Merkmal zur Einstellung der Lagervorspannung ist nach Anspruch 9 vorgesehen, dass der Lagerring zweiteilig ausgebildet ist, wobei dieser mit einer in axialer Richtung verschiebbaren Stellmutter verbunden ist. Dabei hat es sich nach einem weiteren Merkmal gemäß Anspruch 10 als vorteilhaft erwiesen, wenn die Stellmutter über ein Gewinde von einem entsprechenden Gegengewinde des Lagerringes aufgenommen ist.

Schließlich soll nach einem letzten Merkmal der Erfindung das Wälzlager in einem Deckenstativ für medizinische Geräte einsetzbar sein. Derartige Deckenstative sind schon seit längerem bekannt und werden beispielsweise in der DE 36 27 517 A1, DE 43 06 803 A1 und DE 199 63 512 C1 beschrieben. Das in der letzten Vorveröffentlichung beschriebene Deckenstativ ist auch mit einer Bremseinrichtung versehen, die aus zwei Bremsringen besteht, welche die Lageranordnung radial von außen umschließen. Auch hier ist die Bremseinrichtung als zusätzlich zu fertigendes und außerhalb der eigentlichen Lagerung anzuordnendes Bauteil dargestellt, welches wiederum die im Stand der Technik aufgeführten Nachteile beinhaltet.

Weitere Merkmale der Erfindung ergeben sich aus der nachfolgenden Beschreibung und aus den Zeichnungen, in denen Ausführungsbeispiele der Erfindung in vereinfachter Form dargestellt sind.

### Kurze Beschreibung der Zeichnungen

Es zeigen:
- Figur 1: einen Längsschnitt durch ein erfindungsgemäß ausgestaltetes Wälzlager entlang der Linie 1-1 in Figur 2,
- Figur 2: eine Seitenansicht dieses Lagers,
- Figur 3: einen Längsschnitt durch eine zweite Erfindungsvariante und
- Figur 4: einen vergrößerten Ausschnitt aus dem Lager im Bereich der Wälzkörper.

### Ausführliche Beschreibung der Zeichnungen

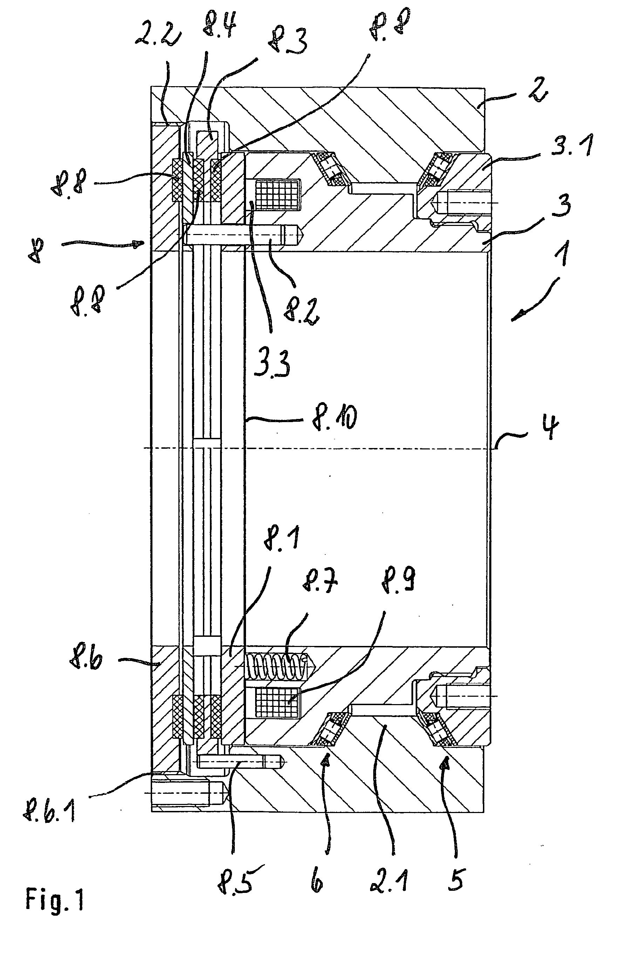

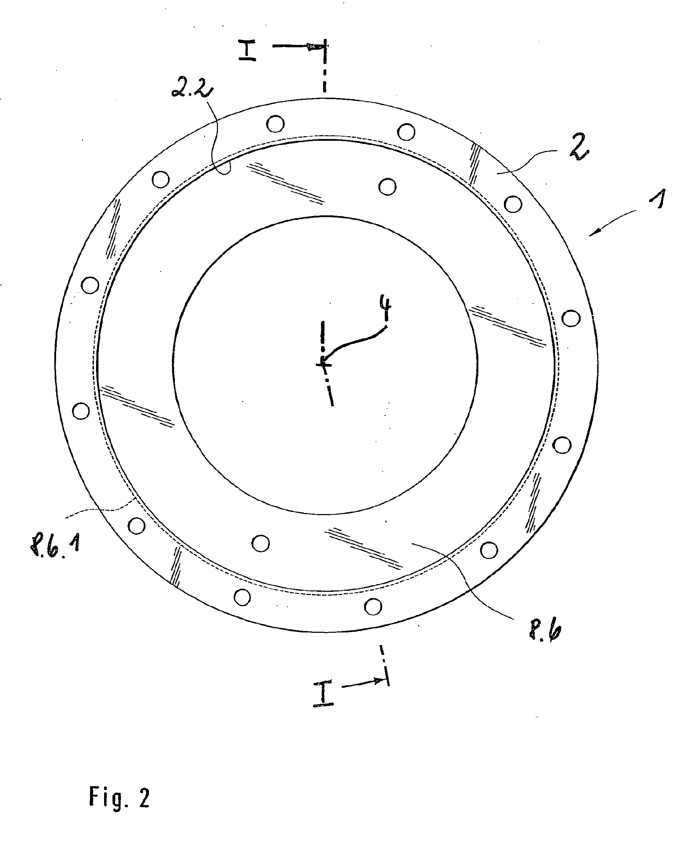

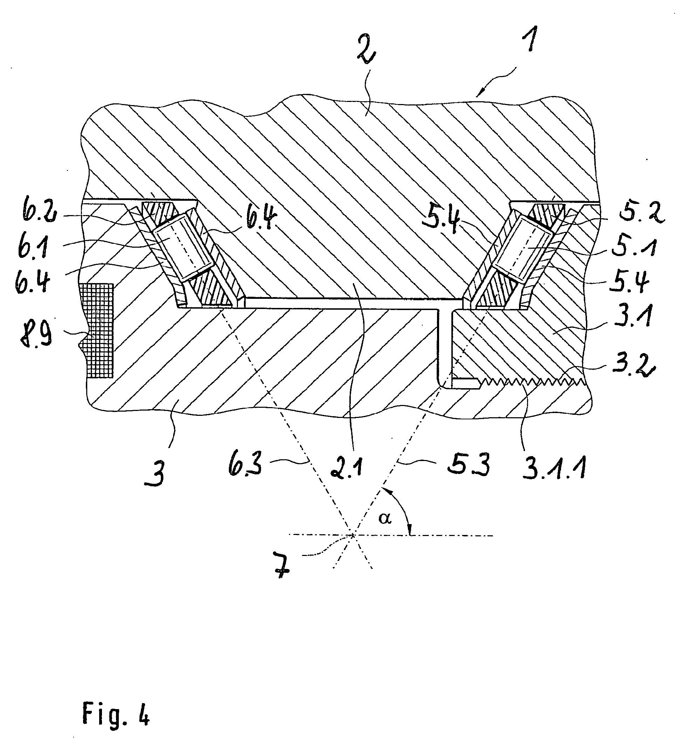

Das in den Figuren 1 bis 4 gezeigte erfindungsgemäß ausgestaltete Wälzlager 1 besteht aus dem Lageraußenring 2 und dem Lagerinnenring 3, die konzentrisch um die Lagerachse 4 ineinander angeordnet sind. Im zwischen beiden gebildeten Ringraum sind Axialschrägnadellager 5, 6 angeordnet, die zueinander in O-Anordnung angestellt sind. Beide weisen Wälzkörper in Form von Lagernadeln 5.1, 6.1 aus, die in je einem Käfig 5.2, 6.2 geführt sind, wobei sich die verlängerten Drehachsen 5.3, 6.3 der Lagemadeln 5.1, 6.2 im Punkt 7 schneiden, der im Lagerinnenring 3 liegt. Zu den Axialschrägnadellagern 5, 6 gehören jeweils zwei Laufscheiben 5.4, 6.4, die die nicht näher bezeichneten Laufbahnen für die Lagernadeln 5.1, 6.1 stellen. Insbesondere die Figur 4 lässt erkennen, dass der Neigungswinkel α veränderbar sein kann und somit das Verhältnis von radialer und axialer Kraftaufnahme beeinflusst werden kann. Wie auch ersichtlich, ist der Lagerinnenring 3 zweiteilig ausgebildet, wobei die Stellmutter 3.1 mit ihrem Innengewinde 3.1.1 auf das Außengewinde 3.2 des Lagerinnenrings 3 aufgeschraubt und damit in axialer Richtung verschiebbar ist. Auf diese Weise kann die Lagervorspannung in einfacher Weise durch Anziehen der Stellmutter 3.1 eingestellt werden, wobei die beiden Axialschrägnadellager 5, 6 gegen den V-förmigen Vorsprung 2.1 des Lageraußenringes 2 gepresst werden.

Nach Figur 1 besteht das erfindungsgemäße Bremselement 8 aus der ferromagnetischen Ankerplatte 8.1, die kreisringartig ausgebildet ist und mit dem rotierenden Lagerinnenring 3 über Führungsstifte 8.2 formschlüssig verbunden ist. Die Führungsstifte 8.2 sind weiter mit der Scheibe 8.4 verbunden, sodass bei rotierendem Lagerinnenring 3 mit diesem die Ankerplatte 8.1 und die Scheibe 8.4 mitrotieren. Die Führungsstifte 8.2 sind in Umfangsrichtung gleichmäßig voneinander beabstandet angeordnet und so dimensioniert, dass sowohl die Ankerplatte 8.1 als auch die Scheibe 8.4 in axialer Richtung verschiebbar sind. Zwischen Ankerplatte 8.1 und Scheibe 8.4 ist eine weitere Scheibe 8.3 angeordnet, die über Führungsstifte 8.5 mit dem feststehenden Lageraußenring 2 verbunden ist. Zum Bremselement 8 gehört weiter die Druckplatte 8.6, die über ihr Gewinde 8.6.1 in das Gewinde 2.2 des Lageraußenringes 2 eingeschraubt ist. Auch gehören gleichmäßig in Umfangsrichtung voneinander beabstandete Federelemente 8.7 dazu, welche die Ankerplatte 8.1 in Richtung der Druckplatte 8.6 pressen. Ankerplatte 8.1, Scheiben 8.3, 8.4 sowie Druckplatte 8.6 sind durch Bremsbeläge 8.8 voneinander getrennt, wobei die Anordnung der Reibpartner so gewählt ist, dass diese abwechselnd drehend und nichtdrehend angeordnet sind. Mit dem Lagerinnenring 3 rotieren über die Führungsstifte 8.2 die Ankerplatte 8.1 und die Scheibe 8.4 mit, während die Druckplatte 8.6 über ihre Gewinde 8.6.1 und die Scheibe 8.3 über die Führungsstifte 8.5 mit dem Lageraußenring 2 festgelegt sind. Die Anordnung der Bremsbeläge 8.8 ist dabei so vorgenommen, dass diese mit der Scheibe 8.3 und der Druckplatte 8.6 fest verbunden sind.

Wie weiter erkennbar, ist der Lagerinnenring 3 mit der in axialer Richtung offenen Ausnehmung 3.3 versehen, in der die Spule 8.9 angeordnet ist. Ankerplatte 8.1 und Lagerinnenring 3 sind im stromlosen Zustand der Spule 8.9 durch den Luftspalt 8.10 voneinander beabstandet, der durch eine unterschiedliche axiale Stellung der Druckplatte 8.6 sehr genau einstellbar ist. Im stromlosen Zustand der Spule 8.9 ist die Lageranordnung gebremst, d. h., der Lagerauβenring 2 und der Lagerinnenring 3 sind reibschlüssig miteinander verbunden. Dabei wird die über die Führungsstifte 8.2 mit dem Lagerinnenring 3 verbundene Ankerplatte 8.1 über den Reibbelag 8.8 gegen die Scheibe 8.3 gepresst, die wiederum über den weiteren Reibbelag 8.8 gegen die Scheibe 8.4 und die wiederum über einen weiteren Bremsbelag 8.8 gegen die Druckplatte 8.6. Bei Stromdurchfluss durch die Spule 8.9 wird durch das erzeugte Magnetfeld die Ankerplatte 8.1 in Richtung Stirnseite des Lagerinnenrings 3 bewegt, sodass diese beiden fest aneinander anliegen und der Luftspalt 8.10 verschwunden ist, sodass der Reibschluss zwischen den Teilen 8.6, 8.4, 8.12, 8.11, 8.3 und 8.1 aufgehoben ist.

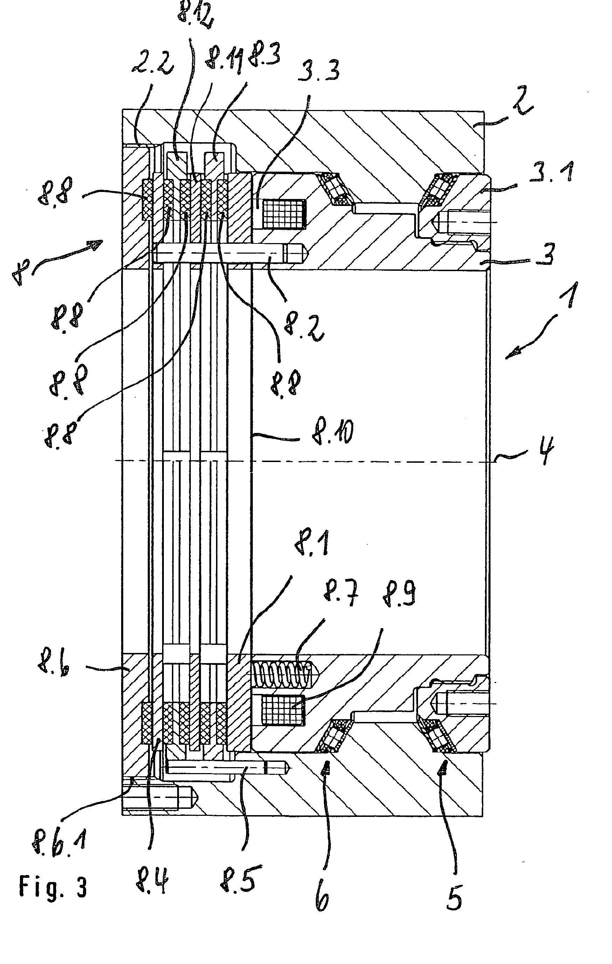

Die in der Figur 3 dargestellte zweite Erfindungsvariante unterscheidet sich von der in Figur 1 gezeigten dadurch, dass zusätzlich zu den Scheiben 8.3 und 8.4 die beiden Scheiben 8.11 und 8.12 angeordnet sind, sodass die Bremsleistung nochmals erhöht ist. Mit dem rotierenden Lagerinnenring 3 sind über die Führungsstifte 8.2 die Ankerplatte 8.1 und die Scheiben 8.4 und 8.11 formschlüssig derart verbunden, sodass sie mitrotieren, aber in axialer Richtung bewegbar sind. Der feststehende Lageraußenring 2 ist über sein Gewinde 2.2 mit dem Gewinde 8.6.1 der Druckplatte 8.6 und über die Führungsstifte 8.5 mit den Scheiben 8.12 und 8.3 verbunden, sodass diese ebenfalls festgelegt sind, wobei die Scheiben 8.12 und 8.3 in axialer Richtung verschiebbar sind. Im stromlosen Zustand der Spule 8.9 ist das Lager 1 gebremst, da über die Federelemente 8.7 die Ankerplatte 8.1, die Scheiben 8.3, 8.11, 8.12, 8.4 gegen die Druckplatte 8.6 gepresst sind, wobei alle beteiligten Partner durch zugehörige Bremsbeläge 8.8 voneinander getrennt sind. Bei Stromfluss hebt die Spule 8.9 die Wirkung der Federelemente 8.7 auf, sodass der Luftspalt 8.10 aufgehoben ist und durch axialen Abstand der beteiligten Partner 8.1, 8.3, 8.11, 8.12, 8.4 und 8.6 zueinander der Reibschluss aufgehoben ist. Die Anordnung der Bremsbeläge 8.8 ist dabei so vorgenommen, dass diese mit den Scheiben 8.3, 8.12 und der Druckplatte 8.6 fest verbunden sind.

### Bezugszeichen

- 1: Wälzlager
- 2: Lageraußenring
- 2.1: Vorsprung
- 2.2: Gewinde
- 3: Lagerinnenring
- 3.1: Stellmutter
- 3.1.1: Innengewinde
- 3.2: Außengewinde
- 3.3: Ausnehmung
- 4: Lagerachse
- 5: Axialschrägnadellager
- 5.1: Lagernadel
- 5.2: Käfig
- 5.3: Drehachse
- 5.4: Laufscheibe
- 6: Axialschrägnadellager
- 6.1: Lagernadel
- 6.2: Käfig
- 6.3: Drehachse
- 6.4: Laufscheibe
- 7: Punkt
- 8: Bremselement
- 8.1: Ankerplatte
- 8.2: Führungsstift
- 8.3: Scheibe
- 8.4: Scheibe
- 8.5: Führungsstift
- 8.6: Druckplatte
- 8.6.1: Gewinde
- 8.7: Federelement
- 8.8: Bremsbelag
- 8.9: Spule
- 8.10: Luftspalt
- 8.11: Scheibe
- 8.12: Scheibe

- α: Neigungswinkel

## Patentansprüche

1. Wälzlager (1) mit einer Bremseinrichtung, insbesondere Drehverbindung, bestehend aus einem Lageraußenring (2) und einem Lagerinnenring (3), zwischen denen auf zugehörigen Laufbahnen Wälzkörper abrollen, wobei zur Erzielung einer Bremswirkung durch Reibschluss ein mit einem der Lagerringe (3,2) verschiebbares Bremselement gegen eine mit dem anderen Lagerring (2, 3) verbundene Gegenfläche gepresst ist und der Reibschluss mit Hilfe eines Elektromagneten aufhebbar ist, **dadurch gekennzeichnet, dass** der Elektromagnet aus einem der Lagerringe (3, 2) als Weicheisenkern und einer diesen umschließenden Spule (8.9) besteht, dass eine mit einem der Lagerringe (3, 2) verbundene ferromagnetische Ankerplatte (8.1) in Richtung einer mit dem anderen Lagerring (2, 3) verbundenen Druckplatte (8.6) gepresst ist und zwischen Ankerplatte (8.1) und Druckplatte (8.6) wenigstens je eine Scheibe (8.4, 8.11, 8.3, 8.12) angeordnet ist, die beidseitig mit Bremsbelägen (8.8) in Wirkverbindung stehen und über getrennt ausgebildete Führungsstifte (8.2, 8.5) mit je einem der Lagerringe (3, 2) formschlüssig verbunden sind.

2. Wälzlager (1) nach Anspruch 1, **dadurch gekennzeichnet, dass** die Ankerplatte (8.1) über mehrere in Umfangsrichtung voneinander beabstandete Führungsstifte (8.2) axial verschiebbar gehalten und über mehrere in Umfangsrichtung voneinander beabstandete Federelemente (8.7) mit einer Vorspannung beaufschlagt ist, wobei im stromlosen Zustand der Spule (8.9) zwischen der Ankerplatte (8.1) und dem Lagerring (3) ein Luftspalt (8.10) gebildet ist.

3. Wälzlager (1) nach Anspruch 1, **dadurch gekennzeichnet, dass** Druckplatte (8.6) kreisringartig ausgebildet ist und über ein Gewinde (8.6.1) von einem zugehörigen Gewinde(2.2) des Lagerringes (2) aufgenommen ist.

4. Wälzlager (1) nach Anspruch 1, **dadurch gekennzeichnet, dass** die Wälzkörper durch Lagernadeln (5.1, 6.1) zweier entgegengerichteter Axialschrägnadellager (5, 6) gebildet sind, wobei ein Schnittpunkt (7) von deren verlängerter Drehachsen (5.3, 6.3) im Lagerinnen- (3) oder im Lageraußenring (2) liegt.

5. Wälzlager (1) nach Anspruch 4, **dadurch gekennzeichnet, dass** die Axialschrägnadellager (5, 6) zueinander in O-Anordnung angestellt sind und Laufbahnen tragende Laufscheiben (5.4, 6.4) aufweisen.

6. Wälzlager (1) nach Anspruch 4, **dadurch gekennzeichnet, dass** die Laufscheiben (5.4, 6.4) einem Härteprozess unterworfen sind.

7. Wälzlager (1) nach Anspruch 4, **dadurch gekennzeichnet, dass** die Laufscheiben (5.4, 6.4) und wenigstens einer der Lagerringe (2, 3) aus unterschiedlichen Werkstoffen gefertigt sind.

8. Wälzlager (1) nach Anspruch 7, **dadurch gekennzeichnet, dass** der spulenlose Lagerring (2) aus einem Leichtmetall oder aus einem Kunststoff gefertigt ist.

9. Wälzlager (1) nach Anspruch 4, **dadurch gekennzeichnet, dass** einer der Lagerringe (3, 2) zur Einstellung der Vorspannung zweiteilig ausgebildet ist, wobei dieser mit einer in axialer Richtung verschiebbaren Stellmutter (3.1) verbunden ist.

10. Wälzlager (1) nach Anspruch 9, **dadurch gekennzeichnet, dass** die Stellmutter (3.1) über ein Gewinde (3.1.1) von einem entsprechenden Gegengewinde (3.2) des Lagerringes (3) aufgenommen ist.

11. Wälzlager (1) nach einem der vorhergehenden Ansprüche **dadurch gekennzeichnet, dass** es in einem Deckenstativ für medizinische Geräte einsetzbar ist.

## Claims

1. Rolling bearing (1) having a braking device, in particular a rotational connection, comprising an outer bearing race (2) and an inner bearing race (3), between which rolling bodies are in rolling motion on associated raceways, wherein in order to achieve a braking effect by frictional engagement a braking element displaceable with one of the bearing races (3, 2) is pressed against an opposing face connected to the other bearing race (2, 3) and the frictional engagement may be canceled by means of an electromagnet, **characterized in that** the electromagnet comprises one of the bearing rings (3, 2) as soft iron core and a coil (8.9) which encloses the former, **in that** a ferromagnetic armature plate (8.1) which is connected to one of the bearing rings (3, 2) is pressed in the direction of a pressure plate (8.6) which is connected to the other bearing ring (2, 3), and at least in each case one disk (8.4, 8.11, 8.3, 8.12) is arranged between the armature plate (8.1) and the pressure plate (8.6), which disks (8.4, 8.11, 8.3, 8.12) are operatively connected on both sides to brake linings (8.8) and are connected positively to in each case one of the bearing rings (3, 2) via locating pins (8.2, 8.5) which are configured separately.

2. Rolling bearing (1) according to Claim 1, **characterized in that** the armature plate (8.1) is held so that it is axially displaceable by multiple, circumferentially spaced locating pins (8.2), and is pre-tensioned by multiple, circumferentially spaced spring elements (8.7), an air gap (8.10) being formed between the armature plate (8.1) and the bearing race (3) in the absence of any current passing through the coil (8.9).

3. Rolling bearing (1) according to Claim 1, **characterized in that** pressure plate (8.6) is of annular design and is received via a thread (8.6.1) by an associated thread (2.2) of the bearing race (2).

4. Rolling bearing (1) according to Claim 1, **characterized in that** the rolling bodies are formed by bearing needle rollers (5.1, 6.1) of two opposing axial angular contact needle-roller bearings (5, 6), a point of intersection (7) of their extended axes of rotation (5.3, 6.3) lying in the inner bearing race (3) or in the outer bearing race (2).

5. Rolling bearing (1) according to Claim 4, **characterized in that** the axial angular contact needle-roller bearings (5, 6) are set in an O-arrangement to one another and have runners (5.4, 6.4) carrying the raceways.

6. Rolling bearing (1) according to Claim 4, **characterized in that** the runners (5.4, 6.4) have been subjected to a hardening process.

7. Rolling bearing (1) according to Claim 4, **characterized in that** the runners (5.4, 6.4) and at least one of the bearing races (2, 3) are made of different materials.

8. Rolling bearing (1) according to Claim 7, **characterized in that** the bearing race (2) without the coil is produced from a light alloy or a plastic.

9. Rolling bearing (1) according to Claim 4, **characterized in that** one of the bearing races (3, 2) for adjustment of the pre-tension is of two-part design, the race being connected to an adjusting nut (3.1) displaceable in an axial direction.

10. Rolling bearing (1) according to Claim 9, **characterized in that** the adjusting nut (3.1) is received via a thread (3.1.1) by a corresponding mating thread (3.2) of the bearing race (3).

11. Rolling bearing (1) according to one of the preceding claims, **characterized in that** it can be used in a ceiling mount for medical appliances.

## Revendications

1. Palier à roulement (1) comprenant un dispositif de freinage, notamment une connexion rotative, constituée d'une bague de palier extérieure (2) et d'une bague de palier intérieure (3), entre lesquelles des corps de roulement roulent sur des pistes de roulement associées, un élément de freinage déplaçable avec l'une des bagues de palier (3, 2) pour produire un effet de freinage par engagement par friction étant pressé contre une surface conjuguée connectée à l'autre bague de palier (2, 3), et l'engagement par friction pouvant être supprimé à l'aide d'un électroaimant, **caractérisé en ce que** l'électroaimant se compose de l'une des bagues de palier (3, 2) en tant que noyau de fer doux et d'une bobine (8.9) entourant celui-ci, **en ce qu'**une plaque d'armature (8.1) ferromagnétique connectée à l'une des bagues de palier (3, 2) est pressée dans la direction d'une plaque de pression (8.6) connectée à l'autre bague de palier (2, 3), et au moins un disque (8.4, 8.11, 8.3, 8.12) est toujours disposé entre la plaque d'armature (8.1) et la plaque de pression (8.6), lequel est en liaison fonctionnelle de chaque côté avec des garnitures de frein (8.8) et est connecté par engagement par coopération de forme à chacune des bagues de palier (3, 2) par le biais de goupilles de guidage (8.2, 8.5) réalisées séparément.

2. Palier à roulement (1) selon la revendication 1, **caractérisé en ce que** la plaque d'armature (8.1) est maintenue de manière déplaçable axialement par le biais de plusieurs goupilles de guidage (8.2) espacées les unes des autres dans la direction périphérique, et est sollicitée avec une précontrainte par le biais de plusieurs éléments de ressort (8.7) espacés les uns des autres dans la direction périphérique, un entrefer (8.10) étant formé dans l'état non alimenté en courant de la bobine (8.9) entre la plaque d'armature (8.1) et la bague de palier (3).

3. Palier à roulement (1) selon la revendication 1, **caractérisé en ce que** la plaque de pression (8.6) est réalisée sous forme de bague circulaire et est reçue par le biais d'un filetage (8.6.1) par un filetage associé (2.2) de la bague de palier (2).

4. Palier à roulement (1) selon la revendication 1, **caractérisé en ce que** les corps de roulement sont formés par des aiguilles de palier (5.1, 6.1) de deux paliers à aiguilles obliques axiaux opposés (5, 6), un point d'intersection (7) entre leurs axes de rotation prolongés (5.3, 6.3) étant situé dans la bague de palier intérieure (3) ou dans la bague de palier extérieure (2).

5. Palier à roulement (1) selon la revendication 4, **caractérisé en ce que** les paliers à aiguilles obliques axiaux (5, 6) sont posés l'un par rapport à l'autre suivant un agencement en O et présentent des disques de roulement (5.4, 6.4) portant des pistes de roulement.

6. Palier à roulement (1) selon la revendication 4, **caractérisé en ce que** les disques de roulement (5.4, 6.4) sont soumis à un processus de durcissement.

7. Palier à roulement (1) selon la revendication 4, **caractérisé en ce que** les disques de roulement (5.4, 6.4) et au moins l'une des bagues de palier (2, 3) sont fabriqués en matériaux différents.

8. Palier à roulement (1) selon la revendication 7, **caractérisé en ce que** la bague de palier (2) sans bobine est fabriquée en un métal léger ou en plastique.

9. Palier à roulement (1) selon la revendication 4, **caractérisé en ce que** l'une des bagues de palier (3, 2) est réalisée en deux parties pour l'ajustement de la précontrainte, celle-ci étant connectée à un écrou de réglage (3.1) déplaçable dans la direction axiale.

10. Palier à roulement (1) selon la revendication 9, **caractérisé en ce que** l'écrou de réglage (3.1) est reçu par le biais d'un filetage (3.1.1) par un filetage conjugué (3.2) correspondant de la bague de palier (3).

11. Palier à roulement (1) selon l'une quelconque des revendications précédentes, **caractérisé en ce qu'**il peut être inséré dans un support au plafond pour appareils médicaux.
